# EUROPEAN PATENT SPECIFICATION

(11) **EP 1 405 613 B1**
(45) Date of publication and mention of the grant of the patent: **22.03.2006**
(21) Application number: 02022172.7
(22) Date of filing: 04.10.2002
(51) Int. Cl.: A61F 2/06

(54) **An implant device for treating aneurisms of the abdominal aorta**
Implantatvorrichtung zur Behandlung von Bauch-Aortenaneurysmen
Implant pour le traitement des anévrismes aortiques

(43) Date of publication of application: 07.04.2004
(73) Proprietor: SORIN BIOMEDICA CARDIO S.R.L., 13040 Saluggia (Vercelli) (IT)
(72) Inventor: Bartorelli, Antonio, 20121 Milano (IT); Vallana, Franco, 10123 Torino (IT)
(74) Representative: Bosotti, Luciano

(56) References cited:
- EP-A- 1 108 400
- WO-A-99/51165
- US-B1- 6 325 823

## Description

The present invention relates to the treatment of aneurysms of the abdominal aorta performed using non-surgical intravascular techniques.

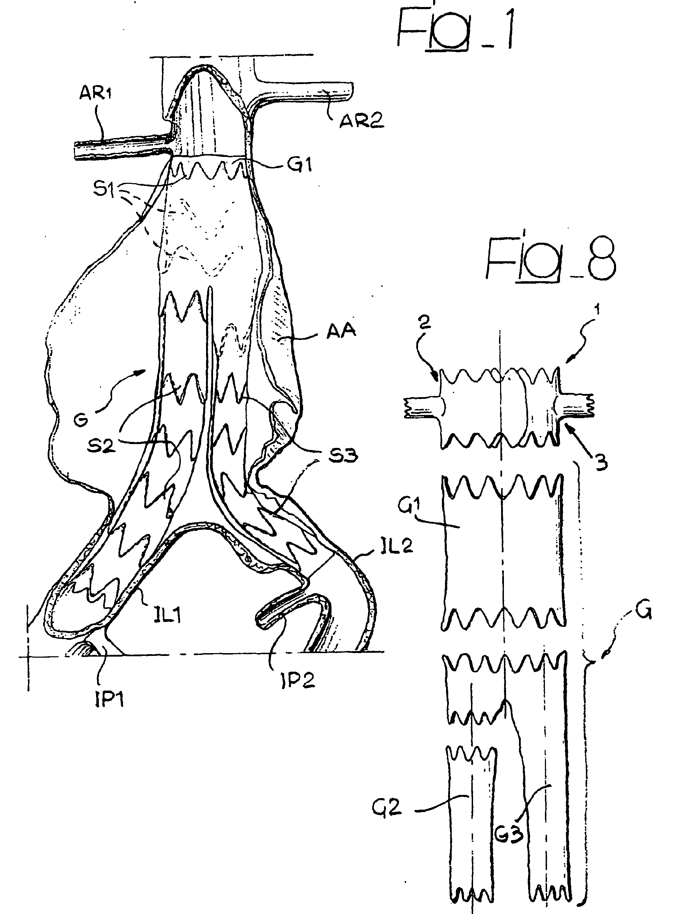

Figure 1 of the annexed drawings is a schematic illustration of a portion of abdominal aorta AA affected by aneurysm.

As may be found in the majority of pathological conditions of this type, the portion in question is the portion of abdominal aorta comprised between the renal arteries AR1 and AR2 and the branching of the iliac arteries IL1 and IL2.

In the above figure, the hypogastric arteries IP1 and IP2 are likewise recognizable, which branch off from the iliac arteries.

The reference G designates, as a whole, an implantation device (of a known type) that can be used for treating the aneurysm described.

Very briefly, the device is an implantation element that has the typical characteristics of an intravascular prosthesis having a general conformation that substantially resembles the shape of a pair of trousers: hence the term "culotte" of French origin sometimes used in the sector.

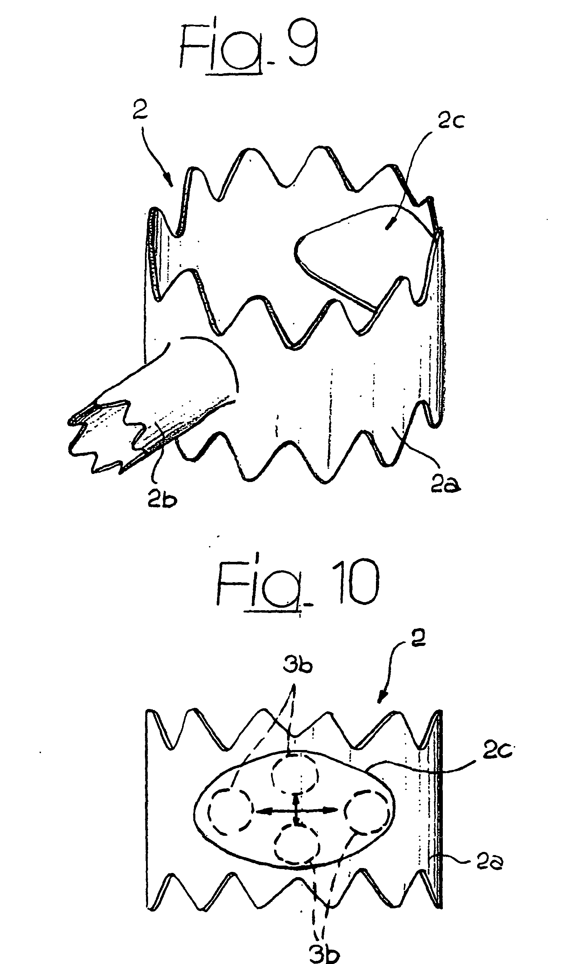

Usually (in this connection, useful reference may be made to Figure 8, which will be described in greater detail hereinafter), the implantation element G comprises a number of parts that can be brought onto the site of implantation by means of a catheterism through the femoral arteries (not illustrated in the drawing) and the iliac arteries IL1, IL2, the aim being to splay out the said implantation element so as to bring the collar part or upper body G1 to anchor to the wall of the abdominal aorta AA in a generically distal position with respect to the renal arteries AR1, AR2 and in a proximal position with respect to the site of the aneurysm. The two "leg" parts of the device G, designated by G2 and G3, are instead located in a position of extension starting from the collar G1 and a position of insertion, respectively, in each of the iliac arteries IL1 and IL2.

As is illustrated more clearly in Figure 8, the various parts G1, G2 and G3 of the implantation element G are usually distinct elements designed to be brought onto the site separately from one another, then to be connected together upon their being spread out so as to bestow on the device G the necessary structural congruence as well as - and this is a factor of extreme importance - the necessary fluid tightness in regard to the blood flow. The aim of the foregoing is to ensure that the blood flow arriving from the upper portion of the aorta can be properly channelled into the iliac arteries IL1, IL2, freeing the walls of the site of the aneurysm from the corresponding pressure load and enabling the possible spontaneous reduction of the site itself or, in any case, preventing the wall of the abdominal aorta AA from being subjected, at said site, to stresses that might cause its failure.

In this connection, it is of particular importance to succeed in ensuring perfect connection and union in conditions of tightness of seal between the proximal margin of the collar part G1 and the wall of the aorta to which the said collar part is anchored. This result can be advantageously obtained by providing the part G1, precisely in the point corresponding to the margin described above, with a supporting element and an anchorage S1 consisting, for example, of an element of the type generally referred to as "stent". Supporting structures of this sort, designated by S2 and S3, are normally provided in other parts G2 and G3 of the device G.

The foregoing description corresponds to notions, principles and criteria of implementation that are altogether known to the prior art and hence are such as not to call for a detailed description herein. In this connection, those of skill in the art will appreciate that the morphology and structure of the implantation device G described previously corresponds to only one of a variety of possible solutions that may be adopted to implement the said method of treatment of aneurysms.

In particular, different variants are possible both as regards the overall structure of the device G and as regards the details of implementation thereof, the presence and the nature of the supporting formations S1, S2, S3, the connection between said supporting structures and the wall of the implantation device, and as regards the technological choices (materials used), as well as - and this latter factor certainly not being of least importance - as regards the modalities adopted for locating the implantation device and/or parts thereof on the implantation site and the subsequent splaying-out in said site, with primary attention being paid to the need to achieve a firm condition of anchorage in the implantation position as well as proper fluid tightness in regard to the blood flow.

Possible phenomena of leakage between the collar part G1 and the corresponding portion of abdominal aorta AA, such as to cause at least part of the blood flow to pass on the outside of the implantation device G, could in fact give rise to situations in which the space between the outer wall of the implantation device G and the inner wall of the portion of aorta subject to aneurysms continues to be fed with blood. The foregoing must be performed in conditions in which the effect of swelling of the site of the aneurysm not only fails to be countered but even tends to be rendered still more evident.

Clinical practice demonstrates the importance and the degree of seriousness of the critical aspects linked to achieving a condition of firm anchorage and fluid tightness at the proximal margin of the implantation device G.

In some cases, the critical nature is linked to the fact that the aneurysm phenomenon involves the wall of the abdominal aorta also close proximity with the ostia of the renal arteries AR1, AR2. In these conditions, there is therefore little space available for achieving the condition of anchorage sought since, as is clearly evident, the implantation device G must not occlude the ostia of the renal arteries.

On the other hand, implantation devices such as the device G just described are produced and made available in pre-determined sizes which do not always fit exactly the anatomical characteristics of the subject undergoing treatment. There thus exists the possibility, for instance, that at least one of the parts G2, G3 will end up extending with its distal margin as far as the ostium of one of the hypogastric arteries IP1, IP2. Albeit not having the serious consequences that could derive from the occlusion of the ostium of one of the renal arteries, this fact does, however, constitute an unfavourable phenomenon that it is desirable to avoid.

It is, therefore, important to be able to offer to the surgeon who carries out the treatment the possibility of intervening on the position of splaying-out and anchorage of the implantation device so as to enable a precise adaptation to the anatomical characteristics of the subject receiving treatment to be achieved. An endovascular prosthesis which is adaptable by means of a telescopie element is disclosed in US-B1-6325823.

The purpose of the present invention is to provide an altogether satisfactory answer to the requirements outlined above.

According to the present invention, the above purpose is achieved thanks to an implantation device having the characteristics referred to specifically in the claims that follow.

The invention will now be described, merely by way of non-limiting example, with reference to the attached drawings, in which:
- Figure 1 has already been described previously to provide a general illustration of the overall context in which the solution according to the invention belongs;
- Figure 2 is a first plan view of an implantation device according to the invention;
- Figure 3 is an exploded perspective view designed to provide a better illustration of the two elements that make up the device according to the invention;
- Figures 4 to 7 are further views designed to illustrate the characteristics and modalities of use of the device according to the invention;
- Figure 8 (which has moreover been referred to previously) illustrates the modalities of possible use of a device according to the invention in conjunction with the implantation device G already described previously; and
- Figures 9 and 10 illustrate a possible variant embodiment of the solution according to the invention.

Basically, the device according to the invention, designated as a whole by 1, can be viewed as a graft (or, in the currently preferred embodiment, a stent-graft) designed to be placed in the implantation condition schematically illustrated by the dashed line in Figure 1, i.e., so as to form a sleeve or lining, working from the inside of the vessel, for the portion of the abdominal aorta that extends in the area corresponding to the ostia of the renal arteries AR1, AR2.

The device 1 according to the invention is therefore designed to constitute a sort of collar or sleeve that is applied on the aforesaid portion of the abdominal aorta in order to provide, at the same time:
- a sufficiently extensive connecting surface between the wall of the abdominal aorta AA and the peripheral wall of the device 1, the aim being to create conditions of fluid tightness such as to prevent any possible risk of leakage in the sense of a possible infiltration of blood flow in the space between the wall of the vessel and the outer wall of the implantation device; and
- a condition of firm longitudinal anchorage, provided also by the shape fit with the ostia of the renal arteries (achieved according to the criteria described in greater detail in what follows), the aim being to create a structure that can enable a firm and complete anchorage of the proximal portion of an implantation device G designed to be fitted to the device 1 according to the invention in accordance with criteria that are illustrated in greater detail in what follows.

It will be appreciated that what has been said above also applies in the case where the phenomenon of aneurysm (or, in general, impairment of the abdominal aorta) extends also in the vicinity of the ostia of the renal arteries.

In other words, the implantation device 1 according to the invention is designed to constitute a sort of support or shoulder aimed at enabling implantation of a device such as the device G described previously to be achieved in any case in an altogether satisfactory way.

The aim of the above is likewise to enable the surgeon who carries out the operation to have at his disposal, as regards the choice of the position of implantation, a more extensive margin as compared to the one allowed in the case where the implantation device G has to be anchored in a position corresponding to its proximal margin, directly to the wall of the vessel being treated.

The device 1 according to the invention is designed to make allowance for an important anatomical factor, given that the morphology of the ostial regions of the renal arteries AR1 and AR2 may present even quite extensive ranges of variation from one individual to another.

The morphological variants in question concern, in the first place, the relative location of the ostia of the renal arteries AR1 and AR2 in a vertical direction, i.e., in the longitudinal direction of the abdominal artery.

As is highlighted in the representation of Figure 1, the two ostia in question are not usually located exactly at the same height, but instead may be staggered with respect to one another by an amount that can reach a maximum value of approximately 3 cm.

In the second place, the origins (ostia) of the two arteries in question may be located both in diametrically opposite regions of the abdominal aorta AA with respect to one another and in regions that form, with respect to one another, an angle that is typically in the range of 180° ± 10-15°, which corresponds to a possible range of variation of approximately 20-30°.

The structure of the device 1 according to the invention takes into account these factors, envisaging that the device 1 will be made up of two distinct elements 2 and 3, which can be fitted together in different, selectively determinable, positions.

As may be appreciated from Figure 3, each of the two elements 2, 3 in question in turn comprises:
- a base part 2a, 3a, having a general band-like conformation and a preferably cylindrical development; and
- a duct part 2b, 3b, which extends in a radial direction and is approximately central with respect to the corresponding body part 2a, 3a.

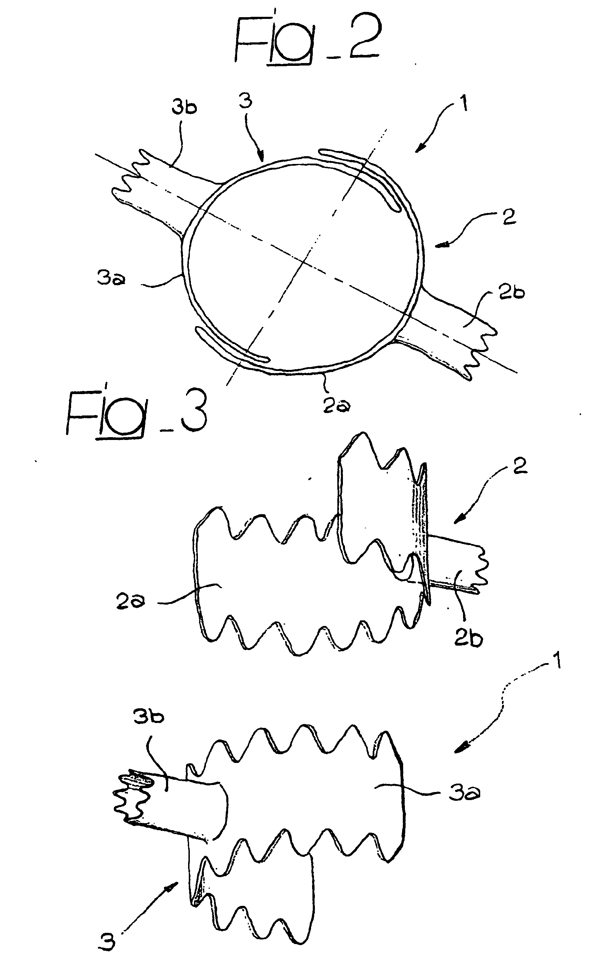

In particular, in the embodiment referred to in Figures 2 to 8, the base part 2a, 3a of the elements 2, 3 has a general C-shaped or fork-like configuration. In this embodiment, the parts 2a and 3a thus present a complete discontinuity in the position generically opposite to the duct part 2b, 3b.

In the variant embodiment to which Figures 9 and 10 refer (where, for reasons of simplicity, just the element 2 has been illustrated), the base part 2a has a ring-like configuration, with a window 2c, which is also located in a position that is roughly opposite to the duct part 2b. Also in this embodiment, the parts 2a and 3a thus present a discontinuity in a position roughly opposite to the duct part 2b, 3b. The difference as compared to the embodiment of Figures 2 to 8 is substantially represented by the fact that, in the case of Figures 9 and 10, the above-mentioned discontinuity is not complete, but only partial.

Whatever the specific embodiment adopted, the two body parts 2a, 3a are preferably made in the form of cylindrical wall portions having practically the same diameter so as to enable their fitting together in a relationship of engagement in conditions of adherence and fluid tightness (in this connection, see, for example, the views presented in Figures 2, 6 and 7), at the same time preserving the possibility of relative orientation in the terms more clearly visible in Figure 7.

In particular, the aforesaid radius or common diameter is chosen in such a way that, in the splayed-out position in the site of the implantation, the two body parts 2a, 3a fitted together jointly define a tubular duct having the diametral dimensions that substantially correspond to those of the portion of abdominal aorta, where the device 1 is placed.

Each of the body parts 2a, 3a is therefore made up of a compliant and (self-)expandable structure and, in the case of the embodiment of Figures 2 to 8, has a general C-shaped conformation, i.e., an open structure.

The device 1 according to the invention consequently demonstrates a considerable capacity of adaptation to the intrinsic elastic characteristics of the wall of the aorta.

As regards the characteristics of compliance mentioned previously, the body parts 2a, 3a are made (according to criteria that are to be deemed on the whole known also as regards the numerous variant embodiments possible), resorting to solutions that substantially resemble the ones adopted for making implantation devices such as the device G described previously, namely as regards the collar part G1 thereof.

The above also applies as regards the possible association to the parts 2a, 3a of a structure that substantially resembles a stent, with the consequent possibility of providing, in regard to the walls of the vessel where the device 1 is implanted, an action of anchorage which amounts to the presence of an elastic load acting in the direction corresponding to radial dilation of the tubular body made up of the parts 2a and 3a fitted together.

In this connection, it will be noted that the proximal and distal margins of both of the parts 2a, 3a have here been illustrated as having a general sinusoidal pattern corresponding to the presence, along the respective end margins, of stent structures (not illustrated in detail, but in themselves known) presenting a typical sinusoidal pattern. The presence of the above reinforcement structures enables, once again according to known criteria, assurance of fit between the two parts 2a, 3a, in the positions illustrated, for example, in Figures 3 and 4 to 7 according to an overall imbricated mechanism of connection.

What has been said previously applies substantially also to the duct parts 2b, 3b. The latter preferably have the typical structure of a sleeve-type graft provided, at the distal margin (i.e., the margin that is to penetrate into the corresponding renal artery), with a corresponding stent structure, which, also here, is illustrated as presenting a sinusoidal pattern. The said stent structure is designed to ensure conditions of firm fluid-tight anchorage of the distal margin of the corresponding duct part 2b, 3b against the wall of the proximal portion of the corresponding renal artery AR1 or AR2.

Also on account of the possible fluid-tight connection and union between the body parts 2a, 3a and the corresponding duct parts 2b, 3b, the considerations already made previously in regard to the possible application of known solutions equally apply here. In this connection, notice, for example, the forked structure of the part G3 of the implantation device G represented in Figure 8.

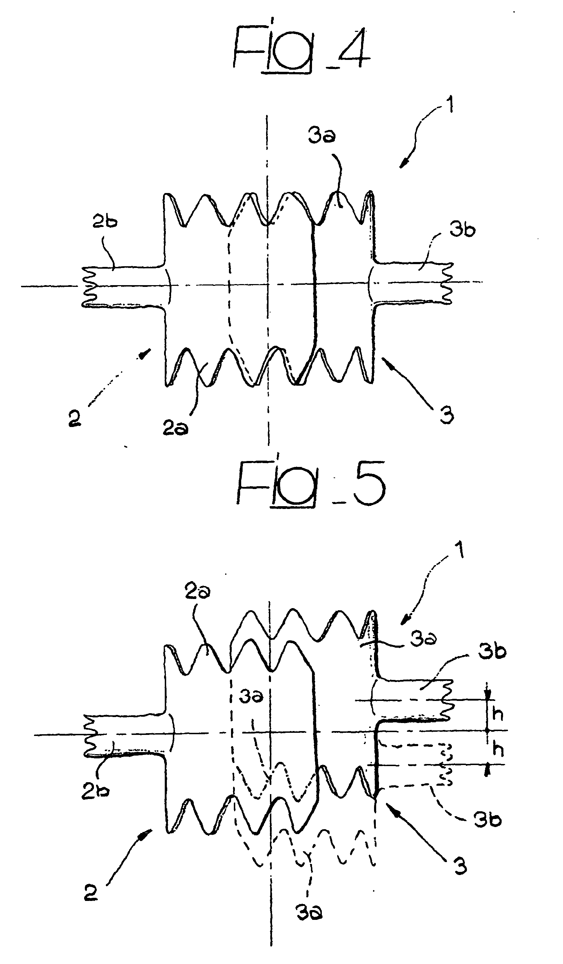

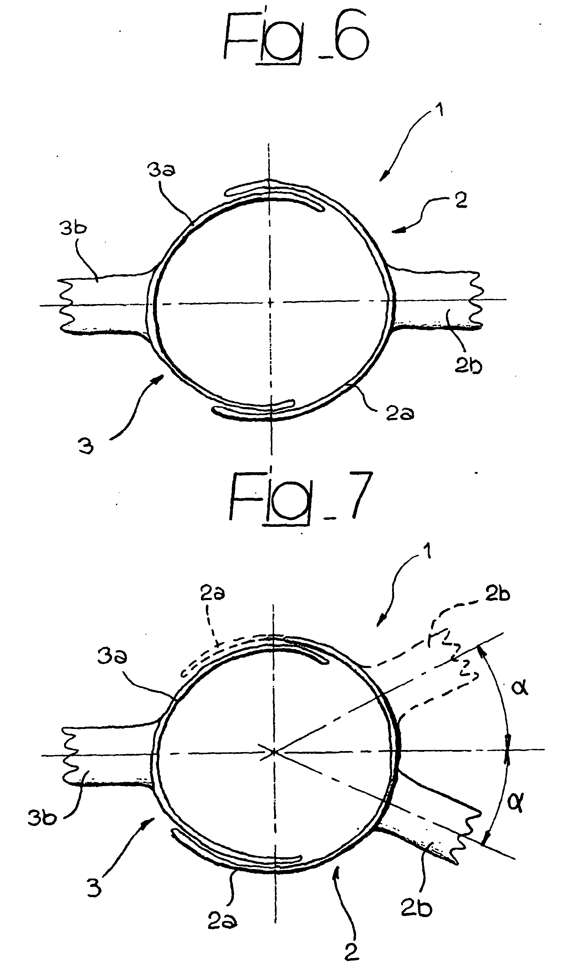

From Figures 4 and 5, it may be realized that the elements 2, 3 of the device 1 may be connected together both maintaining the duct parts 2b, 3b at the same height (see Figure 4) and staggering the height of one with respect to the other (in both directions of possible relative displacement - see, in particular, Figure 5). The aim of this is to enable adaptation to the anatomical morphology of the subject receiving treatment.

Typically, with reference to the situation illustrated in Figure 4, the range of possible relative displacement in height of the two elements 2 and 3 within a range of values between +h and -h is determined according to the normal anatomical data, with a value of h substantially equal to 3 cm approximately.

From Figures 6 and 7, it may be appreciated that the same considerations apply also as regards the relative angular location of the two duct parts 2b, 3b. By selectively modifying the angular position of relative connection of the body parts 2a, 3a, it is possible to pass from a condition in which the two duct parts 2b, 3b are diametrally opposite to one another (Figure 6) to conditions in which the said duct parts are angularly staggered with respect to one another, for instance, in an angular range of typically between 180°±α° (see, in this connection, Figure 7), where α indicates the angle equal to one half of the aforesaid possible range of variation, this angle being, as has already been said, typically between 10° and 15° approximately.

The value of the angle α, which is chosen according to the normal anatomical data, is correlated to the choice of the angular extension of the body part 2a, 3a. This angular extension is chosen so as to have, for example, a value in the region of approximately 320°, so as to ensure that, even in conditions of maximum relative amplitude of angle between the points of origin of the duct parts 2b and 3b, the body parts 2a, 3a will, in any case, be set on top of one another in a relationship of parietal continuity over a very extensive angular range.

In other words, the body parts 2a, 3a present an angular extension, which is interrupted by the solution of continuity characteristic of their general C-shaped configuration, that is at least marginally greater than 180°.

It will be noted from Figures 5 and 7 taken jointly that the two possibilities of relative displacement of the parts 2 and 3, both in height (Figure 5) and in the direction of the relative angular position (Figure 7), can be combined together so as to achieve the desired adaptation to the anatomical characteristics of the subject undergoing treatment.

The diagram of Figure 8 illustrates the modalities with which the device 1 can be associated to an implantation device, such as the implantation device G illustrated previously in order to enable treatment of aneurysm of the abdominal aorta of the type illustrated in Figure 1.

According to known criteria (to which reference has already been made previously) the implantation device G (or usually each of the parts G1, G2, G3 of which the said device is made up) are designed to be placed and spread out on the implantation site by means of catheterism.

A current solution of use envisages that each of the aforementioned parts has a supporting structure or core (the stent) made of superelastic material and/or shape-memory material (this may be, for instance, the material generally known as Nitinol).

Each part of the device G is prepared by encapsulating it in a sheath or tunic that keeps it in contracted conditions, thus allowing it to be mounted on a catheter.

The ensemble thus obtained is used for inserting each part of the device G into the body of the subject being treated, causing it to advance through the vascular system until it reaches the implantation site. At this point, the operator can intervene, usually by acting on a stay or bridle associated to the catheter so as to bring about opening of the sheath that encapsulates the part of the implantation device. Since it is no longer confined by the tunic, the said part expands as a result of the action exerted by the corresponding stent part, to assume the desired configuration.

The same criteria can be adopted for the implantation of the device 1 according to the invention, which, precisely because its primary function is to act as a supporting and anchoring element for the device G, is usually designed to be implanted first in the treated site.

Preferably, the device 1 is implanted in two successive steps, introducing first one and then the other of the elements 2 and 3. Usually, the element introduced first is the element 2, which is designed to be placed in an external position, i.e., to be more precise, to embrace with its body part 2a the homologous body part 3a.

In order to ensure a precise insertion and a firm connection of the duct parts 2b, 3b inside the proximal portions of the renal arteries, it is possible to use catheters having an angled profile (normally referred to as "renal catheters") already currently used for insertion of grafts inside the renal arteries.

The considerations made previously in regard to the embodiment of the invention illustrated in Figures 2 to 8 apply, in a practically identical way, to the variant illustrated in Figures 9 and 10.

Also in this case, the elements 2 and 3 comprise the duct parts 2b and 3b, which are to be implanted in the renal arteries AR1 and AR2. The body parts 2a and 3a, which have an annular shape, can thus be inserted one inside the other by sliding the duct part of one of the elements 2 and 3 in the window provided in the other element. Note, in this connection, the window 2c of the element 2 illustrated in Figures 9 and 10 and the possible insertion therein of the duct part 3b of the element 3.

In other words, in the variant of Figures 9 and 10, each element 2 and 3 comprises:
- a duct part 2b and 3b, designed to be inserted into one of the renal arteries AR1 or AR2; and
- a body part 2a and 3a, provided with a window which is designed to face the other renal artery AR2 or AR1 so as to enable passage of the duct part 3b or 2b of the other element.

The variant of Figures 9 and 10 proves particularly effective from the point of view of exclusion of aneurysm in conditions of fluid tightness in so far as it envisages that both of the body parts 2a and 3a will form a sort of complete collar.

From Figure 10 it may be noticed that the dimensions of the window 2c (and of the homologous window - not illustrated in the drawings - present on the other element 3) are chosen according to the diametral dimensions of the duct parts 2b and 3b, so as to enable the latter to vary their relative positions illustrated with reference to Figures 4 to 7.

In particular, also in the case of the variant embodiment of Figures 9 and 10, the elements 2, 3 of the device 1 can be fitted together, both by keeping the duct parts 2b, 3b at the same height and by staggering them one with respect to the other in height (in either direction of possible relative displacement). This can be obtained thanks to the possibility, offered to each duct part (see, for example, the part 3b illustrated in Figure 10) to move within the window provided in the homologous element (see the window 2c illustrated in Figure 10) in an "axial" direction with respect to the device 1.

The aim of the foregoing is to enable adaptation to the anatomical morphology of the subject undergoing treatment according to the modalities illustrated in Figure 5.

The same considerations apply also as regards relative angular placement of the two duct parts 2b and 3b. In this connection, see once again Figure 10, where there is (also) illustrated the possibility, for the duct part 3b, to shift with respect to the window 2a in a clockwise direction or a counterclockwise direction or in a "tangential" or peripheral direction with respect to the annular development of the body part 2a of the element 2.

Also in this case, it is therefore possible to modify selectively the angular position of relative connection of the body parts 2a, 3a passing from a condition in which the origins or ostia of the two duct parts 2b, 3b are diametrically opposite to one another to conditions in which the said origins are angularly staggered with respect to one another according to the same modalities illustrated in Figure 7.

The above is achieved by exploiting the fact that the body parts 2a, 3a have an angular extension interrupted by the discontinuity represented by the window 2c and by the homologous window present in the element 3 (not specifically illustrated in the drawings), which is at least marginally greater than 180° and typically equal to, for example, 320°. This means, in complementary manner, that also in this case the discontinuity provided in the wall of the body part 2a, 3a has an angular extension in the region of approximately 40°.

Also in the case of the embodiment illustrated in Figures 9 and 10, the possibilities of relative displacement of the parts 2 and 3 both in height and in the direction of relative angular position can be combined together so as to achieve the desired adaptation to the anatomical characteristics of the subject being treated.

Of course, without prejudice to the principle of the invention, the details of construction and the embodiments may vary widely with respect to what is described and illustrated herein, without thereby departing from the scope of the present invention. For example (and without this example necessarily implying any limitation of the possibilities of variation), additional variant embodiments of the invention can certainly be envisaged, in which:
- one of the elements 2, 3 has the structure described with reference to Figures 2 to 8, whilst the other element has the structure described with reference to Figures 9 and 10; or again
- one or both of the elements 2 and 3 are provided with a window part which substantially resembles the window 2c illustrated in Figures 9 and 10, the said window assuming, however, the form of a notch open on one side of the respective base part 2a or 3a.

The latter solution may prove particularly advantageous for the purposes of fitting together the elements 2 and 3, this being obtained by relative axial sliding of said elements.

## Claims

1. An implantation device for the treatment of aneurysms of the abdominal aorta (AA), comprising two complementary elements (2, 3), each of said complementary elements (2, 3) in turn comprising:
- a body part in the form of a band-like part (2a, respectively 3a) that can be connected to the body part (3a, respectively 2a) of the other complementary element so as to give rise to a tubular structure that is able to co-operate with the wall of the abdominal aorta (AA); and **characterised by**
- a duct part (2b, 3b), which extends from the respective body part (2a, 3a), said duct part being insertable into the proximal portion of a renal artery (AR1 and AR2).

2. The implantation device according to Claim 1, **characterized in that**, in at least one of said complementary elements (2, 3), said body part (2a, 3a) exhibits a complete discontinuity according to a general C-shaped conformation.

3. The implantation device according to Claim 1 or Claim 2, **characterized in that**, in at least one of said complementary elements (2, 3), said body part (2a, 3a) exhibits a partial discontinuity defining, within the respective body part 2, 3, a window-like formation.

4. The implantation device according to Claim 3, **characterized in that** said window-like formation (2c) is a closed formation.

5. The implantation device according to Claim 3, **characterized in that** said window-like formation (2c) is a formation open on one side of the respective body part.

6. The implantation device according to any one of the preceding claims, **characterized in that** the body parts (2a, 3a) of said complementary elements (2, 3) develop according to cylindrical surfaces.

7. The implantation device according to Claim 2 or Claim 3, **characterized in that** said body parts (2a, 3a) present an angular extension, interrupted by said discontinuity, which is at least marginally greater than 180°.

8. The implantation device according to Claim 7, **characterized in that** said angular extension is in the region of approximately 320°.

9. The implantation device according to any one of the preceding claims, **characterized in that** said duct parts (2b, 3b) are placed in a substantially central position with respect to the corresponding body parts (2a, 3a) so that said complementary elements (2, 3) can be connected together both in conditions in which said duct parts (2b, 3b) are diametrically opposite to one another and in conditions in which said duct parts (2b, 3b) are angularly staggered with respect to one another.

10. The implantation device according to any one of the preceding claims, **characterized in that** said body parts (2a, 3a) can be selectively connected together in positions of relative longitudinal staggering with respect to the direction of extension of said tubular structure.

11. The implantation device according to Claim 10, **characterized in that** said body parts (2a, 3a) are sized in such a way as to enable a relative longitudinal staggering between said two complementary elements (2, 3) in a range roughly of ±3 cm.

12. The implantation device according to any one of the preceding claims, **characterized in that** said body parts (2a, 3a) carry associated stent-type supporting structures.

13. The implantation device according to any one of the preceding claims, **characterized in that** it comprises respective stent-type supporting structures associated to the distal margins of said duct parts (2b, 3b).

14. The implantation device according to any one of the preceding claims, **characterized in that** said body parts (2a, 3a) are configured in such a way that, in the connecting position, said tubular structure constitutes a supporting and anchoring structure for a further implantation device (G) for channelling of an aneurysm of the abdominal aorta.

## Patentansprüche

1. Implantat zur Behandlung von Aneurysmen der abdominalen Aorta (AA), mit zwei komplementären Elementen (2, 3), wobei jedes der beiden komplementären Elemente (2, 3) wiederum beinhaltet:
- einen Körperteil in der Form eines bandförmigen Teils (2a, bzw. 3a), der mit dem Körperteil (3a, bzw. 2a) des anderen komplementären Elements so verbunden werden kann, dass eine röhrenförmige Struktur entsteht, die in der Lage ist, mit der Wand der abdominalen Aorta (AA) zusammen zu arbeiten; und **gekennzeichnet durch**
- einen Röhrenteil (2b, 3b), der sich von dem jeweiligen Körperteil (2a, 3a) erstreckt, wobei der Röhrenteil zum Einführen in den proximalen Bereich einer renalen Arterie (AR1 und AR2) geeignet ist.

2. Implantat nach Anspruch 1, **dadurch gekennzeichnet, dass** zumindest bei einem der beiden komplementären Elemente (2, 3) der Körperteil (2a, 3a) eine vollständige Diskontinuität bezüglich einer allgemeinen C-förmigen Konformation aufweist.

3. Implantat nach Anspruch 1 oder Anspruch 2, **dadurch gekennzeichnet, dass** zumindest bei einem der komplementären Elemente (2, 3) der Körperteil (2a, 3a) eine partielle Diskontinuität aufweist, die in dem jeweiligen Körperteil (2, 3) ein fensterartiges Gebilde definiert.

4. Implantat nach Anspruch 3, **dadurch gekennzeichnet, dass** das fensterartige Gebilde (2c) ein geschlossenes Gebilde ist.

5. Implantat nach Anspruch 3, **dadurch gekennzeichnet, dass** das fensterartige Gebilde (2c) ein Gebilde ist, das an einer Seite des jeweiligen Körperteils offen ist.

6. Implantat nach einem der vorhergehenden Ansprüche, **dadurch gekennzeichnet, dass** die Körperteile (2a, 3a) der komplementären Elemente (2, 3) sich entsprechend zylindrischen Oberflächen entwickeln.

7. Implantat nach Anspruch 2 oder 3, **dadurch gekennzeichnet, dass** die Körperteile (2a, 3a) eine Winkelerstreckung, die von der Diskontinuität unterbrochen wird, aufweisen, die zumindest geringfügig größer als 180° ist.

8. Implantat nach Anspruch 7, **dadurch gekennzeichnet, dass** die Winkelerstreckung in dem Bereich von ungefähr 320° liegt.

9. Implantat nach einem der vorhergehenden Ansprüche, **dadurch gekennzeichnet, dass** die Röhrenteile (2b, 3b) mit Bezug auf die entsprechenden Körperteile (2a, 3a) in einer im wesentlichen zentralen Position angeordnet sind, so dass die komplementären Elemente (2, 3) sowohl unter den Bedingungen, dass die Röhrenteile (2b, 3b) sich diametral gegenüberliegen, als auch unter den Bedingungen, dass die Röhrenteile (2b, 3b) winklig gestaffelt zueinander angeordnet sind, miteinander verbunden werden können.

10. Implantat nach einem der vorhergehenden Ansprüche, **dadurch gekennzeichnet, dass** die Körperteile (2a, 3a) selektiv miteinander in Positionen relativer Längsstaffelung, mit Bezug auf die Richtung der Erstreckung der röhrenförmigen Struktur, verbunden werden können.

11. Implantat nach Anspruch 10, **dadurch gekennzeichnet, dass** die Körperteile (2a, 3a) so bemessen sind, dass sie eine relative Längsstaffelung zwischen den zwei komplementären Elementen (2, 3) in einem Bereich von ungefähr ± 3 cm ermöglichen.

12. Implantat nach einem der vorhergehenden Ansprüche, **dadurch gekennzeichnet, dass** die Körperteile (2a, 3a) assoziierte Unterstützungsstrukturen vom Stent-Typ tragen.

13. Implantat nach einem der vorhergehenden Ansprüche, **dadurch gekennzeichnet, dass** es jeweils Stent-Typ artige Unterstützungsstrukturen aufweist, die den distalen Rändern der Röhrenteile (2b, 3b) zugehörig sind.

14. Implantat nach einem der vorhergehenden Ansprüche, **dadurch gekennzeichnet, dass** die Körperteile (2a, 3a) in der Weise ausgeführt sind, dass in der verbundenen Position die röhrenförmige Struktur eine unterstützende und eine verankernde Struktur für ein weiteres Implantat (G) für die Kanalisierung eines Aneurysmas der abdominalen Aorta bildet.

## Revendications

1. Dispositif d'implantation pour le traitement d'anévrismes de l'aorte abdominale (AA), comprenant deux éléments complémentaires (2, 3), chacun desdits éléments complémentaires (2, 3) comprenant à son tour :
- une partie de corps sous la forme d'une partie en bande (2a, respectivement 3a) qui peut être raccordée à la partie de corps (3a, respectivement 2a) de l'autre élément complémentaire de manière à produire une structure tubulaire qui est capable de coopérer avec la paroi de l'aorte abdominale (AA); et **caractérisé par**
- une partie de conduit (2b, 3b), qui s'étend de la partie de corps respective (2a, 3a), ladite partie de conduit pouvant être insérée dans la partie proximale d'une artère rénale (AR1 et AR2).

2. Dispositif d'implantation selon la revendication 1, **caractérisé en ce que**, dans au moins l'un desdits éléments complémentaires (2, 3), ladite partie de corps (2a, 3a) présente une complète discontinuité selon une conformation générale en forme de C.

3. Dispositif d'implantation selon la revendication 1 ou la revendication 2, **caractérisé en ce que**, dans au moins l'un desdits éléments complémentaires (2, 3), ladite partie de corps (2a, 3a) présente une discontinuité partielle définissant, dans la partie de corps respective 2, 3, une structure en forme de fenêtre.

4. Dispositif d'implantation selon la revendication 3, **caractérisé en ce que** ladite structure en forme de fenêtre (2c) est une structure fermée.

5. Dispositif d'implantation selon la revendication 3, **caractérisé en ce que** ladite structure en forme de fenêtre (2c) est une structure ouverte sur un côté de la partie de corps respective.

6. Dispositif d'implantation selon l'une quelconque des revendications précédentes, **caractérisé en ce que** les parties de corps (2a, 3a) desdits éléments complémentaires (2, 3) se développent selon des surfaces cylindriques.

7. Dispositif d'implantation selon la revendication 2 ou la revendication 3, **caractérisé en ce que** lesdites parties de corps (2a, 3a) présentent une extension angulaire, interrompue par ladite discontinuité, qui est au moins marginalement supérieure à 180°.

8. Dispositif d'implantation selon la revendication 7, **caractérisé en ce que** ladite extension angulaire se situe dans la région d'environ 320°.

9. Dispositif d'implantation selon l'une quelconque des revendications précédentes, **caractérisé en ce que** lesdites parties de conduit (2b, 3b) sont placées en position sensiblement centrale par rapport aux parties de corps correspondantes (2a, 3a) de sorte que lesdits éléments complémentaires (2, 3) puissent être raccordés ensemble à la fois dans des conditions dans lesquelles lesdites parties de conduit (2b, 3b) sont diamétralement opposées l'une à l'autre et dans des conditions dans lesquelles lesdites parties de conduit (2b, 3b) présentent un étagement angulaire l'une par rapport à l'autre.

10. Dispositif d'implantation selon l'une quelconque des revendications précédentes, **caractérisé en ce que** lesdites parties de corps (2a, 3a) peuvent sélectivement être raccordées ensemble dans des positions d'étagement longitudinal relatif par rapport à la direction d'extension de ladite structure tubulaire.

11. Dispositif d'implantation selon la revendication 10, **caractérisé en ce que** lesdites parties de corps (2a, 3a) sont dimensionnées de manière à permettre un étagement longitudinal relatif entre lesdits deux éléments complémentaires (2, 3) dans une plage d'environ ± 3 cm.

12. Dispositif d'implantation selon l'une quelconque des revendications précédentes, **caractérisé en ce que** les parties de corps (2a, 3a) portent des structures de support associées de type stent.

13. Dispositif d'implantation selon l'une quelconque des revendications précédentes, **caractérisé en ce qu'**il comprend des structures de support respectives de type stent associées aux marges distales desdites parties de conduit (2b, 3b).

14. Dispositif d'implantation selon l'une quelconque des revendications précédentes, **caractérisé en ce que** lesdites parties de corps (2a, 3a) sont configurées de sorte que, en position de raccordement, ladite structure tubulaire constitue une structure de support et d'ancrage pour un autre dispositif d'implantation (G) pour canaliser un anévrisme de l'aorte abdominale.
